# EUROPÄISCHE PATENTSCHRIFT

(11) **EP 3 092 054 B1**
(45) Veröffentlichungstag und Bekanntmachung des Hinweises auf die Patenterteilung: **25.10.2017**
(21) Anmeldenummer: 15700058.9
(22) Anmeldetag: 07.01.2015
(51) Int. Cl.: B01D 53/14, B01D 3/14, B01D 5/00, C07C 41/42

(54) **TRENNTECHNISCHE BEARBEITUNG EINES AUS EINEM PRODUKTSTROM EINES DIMETHYLETHERREAKTORS GEBILDETEN GASGEMISCHS**
SEPARATION OF A GAS MIXTURE FORMED FROM A FLOW OF PRODUCTS OF A DIMETHYL ETHER REACTOR
TRAITEMENT DE SÉPARATION D'UN MÉLANGE GAZEUX CONSTITUÉ D'UN FLUX DE PRODUIT D'UN RÉACTEUR À DIMÉTHYLÉTHER

(30) Priorität: 07.01.2014 EP 14000042
(43) Veröffentlichungstag der Anmeldung: 16.11.2016
(73) Patentinhaber: Linde Aktiengesellschaft, 80331 München (DE)
(72) Erfinder: FRITZ, Helmut, 81375 München (DE); PESCHEL, Andreas, 82515 Wolfratshausen (DE)
(86) Internationale Anmeldenummer: PCT/EP2015/050166
(87) Internationale Veröffentlichungsnummer: WO 2015/104290

(56) Entgegenhaltungen:
- US-A- 5 908 963
- US-A1- 2009 156 698
- FANG D ET AL: "Preparation of dimethyl ether involves condensing reaction product, absorption, rectification of dimethyl ether, rectification of methanol, and preparation of dimethyl ether by dehydrating methanol", WPI / THOMSON,, Bd. 2010, Nr. 9, 23. Dezember 2009 (2009-12-23), XP002725398, & CN 101 607 873 A (UNIV EAST CHINA SCI&TECHNOLOGY) 23. Dezember 2009 (2009-12-23)

## Beschreibung

Die Erfindung betrifft ein Verfahren zur trenntechnischen Bearbeitung eines Gasgemischs, das aus einem Produktstrom eines Reaktors zur Synthese von Dimethylether aus Synthesegas gebildet wird.

### Stand der Technik

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether kann entweder direkt aus Methanol oder indirekt aus Erd- oder Biogas erzeugt werden. Im letzteren Fall wird das Erd- oder Biogas zunächst zu Synthesegas reformiert. Synthesegas kann auch mittels anderer Verfahren, beispielsweise durch Pyrolyse von Abfällen oder Biomasse, gewonnen werden. Das Synthesegas wird anschließend entweder in einer zweistufigen Reaktion zu Methanol und anschließend zu Dimethylether oder in einer einstufigen Reaktion direkt zu Dimethylether konvertiert.

Die Synthese von Dimethylether aus Synthesegas ist thermodynamisch und wirtschaftlich vorteilhaft gegenüber einer Synthese aus Methanol.

Die vorliegende Erfindung betrifft insbesondere die einstufige Synthese von Dimethylether, wobei unter einer "einstufigen" Synthese eine Synthese verstanden wird, bei der sämtliche Reaktionen in einunddemselben Reaktor ablaufen. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 1 00 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor erhaltener Produktstrom wird kopfseitig entnommen, gekühlt und einer Trennung zugeführt.

Der Produktstrom enthält neben Dimethylether nicht umgesetzte Komponenten des Synthesegases sowie weitere Reaktionsprodukte. Typischerweise weist der Produktstrom neben Dimethylether zumindest Methanol, Wasser, Kohlendioxid, Kohlenmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlendioxid und leichter als Kohlendioxid siedende Komponenten wie Wasserstoff und Kohlenmonoxid enthalten. Zur spezifikationsgerechten Gewinnung von Dimethylether müssen diese zumindest teilweise abgetrennt werden. Hierzu verwendete Verfahren erweisen sich jedoch insbesondere unter energetischen Aspekten als unbefriedigend.

Zur Gewinnung von Dimethylether aus dem Produktstrom muss dieser auf Temperaturen deutlich unter 0 °C abgekühlt werden. Hierbei kann es erforderlich sein, vor der Abkühlung größere Mengen an Methanol und Wasser abzutrennen. Die vorliegende Erfindung zeigt jedoch auch Verfahren auf, bei denen eine derartige Abtrennung nicht erforderlich ist.

Aus der US 5 908 963 A, auf der der Oberbegriff von Anspruch 1 beruht, ist ein Verfahren zur Herstellung eines Dimetyletherprodukts bekannt, bei dem ein aus einem Produktstrom eines Dimethyletherreaktors in einer Destillationssäule ein Methanol und Wasser enthaltendes Gemisch auskondensiert wird. Aus der verbleibenden Gasphase wird Dimethylether mit dem aus diesem Gemisch gewonnenen Methanol ausgewaschen.

Es besteht insgesamt der Bedarf nach verbesserten Möglichkeiten zur trenntechnischen Bearbeitung entsprechender Gasgemische.

### Offenbarung der Erfindung

Vor diesem Hintergrund schlägt die vorliegende Erfindung ein Verfahren zur trenntechnischen Bearbeitung eines Gasgemischs, das aus einem Produktstrom eines Reaktors zur Synthese von Dimethylether aus Synthesegas gebildet wird, und das zumindest Dimethylether, Kohlendioxid und zumindest eine weitere, leichter als Kohlendioxid siedende Komponente enthält, gemäß den Merkmalen des unabhängigen Patentanspruchs vor. Bevorzugte Ausführungsformen sind Gegenstand der Unteransprüche sowie der nachfolgenden Beschreibung.

Vor der Erläuterung der Merkmale und Vorteile der vorliegenden Erfindung werden deren Grundlagen und die verwendeten Begriffe erläutert.

Ein Fluid (die Bezeichnung "Fluid" wird nachfolgend auch kurz für entsprechende Ströme, Fraktionen usw. verwendet) ist von einem anderen Fluid (das auch als Ausgangsfluid bezeichnet wird) "abgeleitet" oder aus einem solchen Fluid "gebildet", wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Strom kann auch beispielsweise einfach dadurch "gebildet" werden, dass er aus einem Speicherbehälter abgezogen wird.

Fluide können im hier verwendeten Sprachgebrauch reich oder arm an einer oder mehreren enthaltenen Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Sie können im hier verwendeten Sprachgebrauch angereichert oder abgereichert an einer oder mehreren Komponenten sein, wobei sich diese Begriffe auf einen entsprechenden Gehalt in einem Ausgangsfluid beziehen, aus dem das Fluid gebildet wurde. Das Fluid ist "angereichert", wenn es zumindest den 1,1-fachen, 1,5-fachen, 2-fachen, 5-fachen, 10-fachen 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsfluid, enthält. Ein "überwiegend" eine oder mehrere Komponenten enthaltendes Fluid enthält diese eine oder mehreren Komponenten zu wenigstens 90%, 95%, 98% oder 99% bzw. ist reich an diesen.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte verwendet werden müssen, um ein erfinderisches Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Unterschiedliche Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Fluid) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert oder reich bzw. arm im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, die hier als Sumpfflüssigkeit bezeichnet wird, wird in einer Destillationssäule mittels eines Sumpfverdampfers erwärmt, so dass kontinuierlich ein Teil der Sumpfflüssigkeit verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, hier als Kopfgas bezeichnet, eingespeist, zu einem Teil zu einem Kondensat verflüssigt und als flüssiger Rücklauf am Kopf der Destillationssäule aufgegeben wird. Ein Teil des aus dem Kopfgas erhaltenen Kondensats kann anderweitig verwendet werden.

Im Gegensatz zu einer Destillationssäule verfügt eine "Absorptionskolonne" typischerweise nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. Die Gasphase wird mit der Lösungsphase "gewaschen". In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen. Am Kopf einer solchen Absorptionskolonne wird ein gasförmiges Fluid erhalten, das als "Kopfprodukt" aus dieser abgezogen werden kann. Im Sumpf der Absorptionskolonne scheidet sich eine Flüssigkeit ab, die als "Sumpfprodukt" abgezogen werden kann. Die Gasphase wird in der Absorptionskolonne bezüglich einer oder mehrerer Komponenten abgereichert, welche in das Sumpfprodukt übergehen.

Zur Auslegung und spezifischen Ausgestaltung von Destillationssäulen und Absorptionskolonnen sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

Ist nachfolgend kurz von einer "Synthese" von Dimethylether die Rede, wird hierunter ein Verfahren verstanden, bei dem ein Synthesegas enthaltender Einsatz, also ein Gasgemisch, das in geeigneten Anteilen zumindest Kohlenmonoxid und Wasserstoff enthält, zu einem entsprechenden Dimethylether enthaltenden Produktstrom umgesetzt wird. Ein entsprechender Produktstrom enthält aufgrund der nicht vollständigen Reaktion und aufgrund des Auftretens von Nebenreaktionen bei der Synthese von Dimethylether, insbesondere in Abhängigkeit von den Charakteristika der verwendeten Katalysatoren und den jeweiligen Gehalten der Komponenten des Synthesegases, nicht ausschließlich Dimethylether sondern weitere Verbindungen. Diese sind zumindest Methanol, Wasser, Kohlendioxid, Kohlenmonoxid und Wasserstoff, jedoch typischerweise auch geringere Mengen Methan, Ethan, organische Säuren und höhere Alkohole. Diese genannten weiteren Verbindungen müssen, wie erwähnt, abgetrennt werden. Die Abtrennung erfolgt einerseits, um nachfolgende Trennschritte zu ermöglichen, und andererseits, um Dimethylether in der geforderten Reinheit, also "spezifikationsgerecht" zu gewinnen.

### Vorteile der Erfindung

In dem erfindungsgemäßen Verfahren wird, wie erläutert, ein Gasgemisch trenntechnisch bearbeitet, das aus einem Produktstrom eines Reaktors zur Synthese von Dimethylether aus Synthesegas gebildet wird, und das zumindest Dimethylether, Kohlendioxid und zumindest eine weitere, leichter als Kohlendioxid siedende Komponente enthält. Bei solchen, leichter als Kohlendioxid siedenden Komponenten kann es sich insbesondere um Komponenten wie Kohlenmonoxid und Wasserstoff handeln. Wie erwähnt, sind andere, ebenfalls leichter als Kohlendioxid siedende Komponenten, beispielsweise Methan, in einem derartigen Gasgemisch in geringerer Menge ebenfalls enthalten.

Eine "leichter als Kohlendioxid siedende" Komponente besitzt einen niedrigeren Siedepunkt als Kohlendioxid. Es sei an dieser Stelle darauf hingewiesen, dass bei den erfindungsgemäß eingesetzten Druckniveaus (das nachfolgend erläuterte "erste" Druckniveau liegt oberhalb des Tripelpunkts von Kohlendioxid) Kohlendioxid auch in der flüssigen Phase vorliegen kann.

Dabei wird das Gasgemisch auf einem ersten Druckniveau ausgehend von einem ersten Temperaturniveau auf ein zweites Temperaturniveau abgekühlt. Dies kann vorteilhafterweise über ein oder mehrere Zwischentemperaturniveaus und unter Abscheidung eines oder mehrerer Kondensate aus dem Gasgemisch erfolgen, es ist jedoch auch eine einstufige Abkühlung möglich.

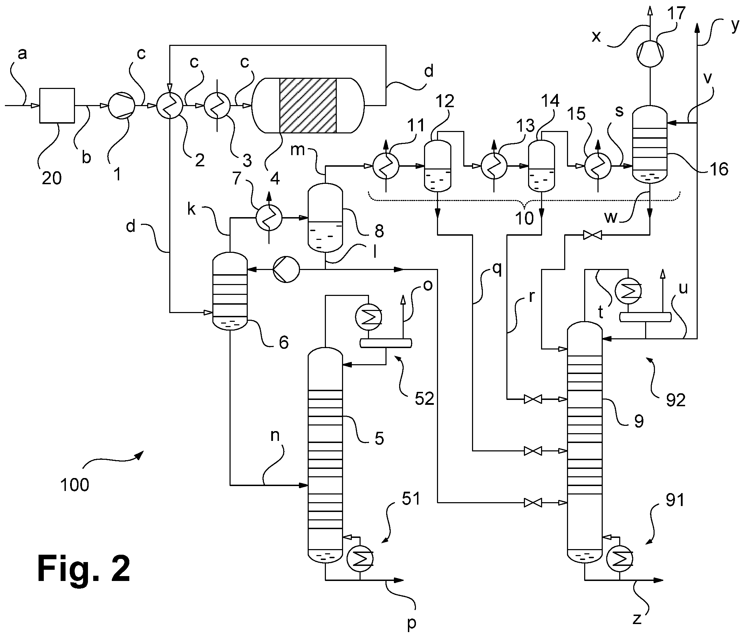

Wenn zuvor Kondensate abgeschieden werden, enthalten diese überwiegend Dimethylether und Kohlendioxid, wenn das verwendete Gasgemisch arm an schwerer siedenden Komponenten wie Methanol und Wasser ist. Beispielsweise kann das Gasgemisch frei oder weitgehend frei von Methanol und Wasser sein, weil Methanol und Wasser zuvor abgetrennt werden. Hierzu kann beispielsweise eine Anordnung zum Einsatz kommen, wie sie in der Figur 2 gezeigt ist. Alternativ dazu kann auf eine Abtrennung verzichtet werden. Wird auf eine Abtrennung verzichtet, ist jedoch darauf zu achten, dass weder Methanol noch Wasser alleine in einem entsprechenden Gasgemisch enthalten sind. Wasser ohne einen zusätzlichen Anteil Methanol würde bei den eingesetzten tiefen Temperaturen ausfrieren, weil die Frostschutzwirkung des Methanols fehlen würde. Umgekehrt würde jedoch auch "trockenes" Methanol zu Nachteilen führen, weil dieses die verwendeten Wärmetauscher schädigen könnte.

Ein auf dem zweiten Temperaturniveau gasförmig bleibender Anteil des Gasgemischs wird in einer Absorptionskolonne unter Gewinnung eines Kopfprodukts und eines Sumpfprodukts mit einem erfindungsgemäß überwiegend Kohlendioxid enthaltenden Rücklauf gewaschen. Dies dient dazu, in dem gasförmig bleibenden Anteil des Gasgemischs noch enthaltenen Dimethylether in das Sumpfprodukt auszuwaschen, so dass das Kopfprodukt nach Möglichkeit weitgehend frei von Dimethylether ist. Das Kopfprodukt besteht überwiegend aus Kohlendioxid und der zumindest einen auf dem ersten Druckniveau leichter als Kohlendioxid siedenden Komponente. Das Kopfprodukt enthält vorzugsweise keinen Dimethylether oder allenfalls geringe Mengen, ist also zumindest arm an Dimethylether im oben erläuterten Sinn.

Der überwiegend Kohlendioxid enthaltende Rücklauf wird aus einem bei der Abkühlung auf das zweite Temperaturniveau flüssig abgeschiedenen Anteil des Gasgemischs gebildet. Vorzugsweise wird hierzu eine Dimethylether-Kohlendioxid-Destillationssäule verwendet. Werden bei der Abkühlung ein oder mehrere Kondensate abgeschieden, werden diese oder aus diesen gebildete Ströme vorzugsweise zumindest zum Teil in die Dimethylether-Kohlendioxid-Destillationssäule eingespeist. In die Dimethylether-Kohlendioxid-Destillationssäule kann auch ein Sumpfprodukt aus der Absorptionskolonne oder ein hieraus gebildeter Strom zumindest zum Teil eingespeist werden.

Unter einer "Dimethylether-Kohlendioxid-Destillationssäule" wird hier eine Destillationssäule verstanden, die so ausgebildet ist und betrieben wird, dass sich in ihr aus Fluiden, die Dimethylether und Kohlendioxid enthalten, Fluide gewinnen lassen, die einerseits an Dimethylether und andererseits an Kohlendioxid angereichert und an der jeweils anderen Komponente abgereichert sind. Der Fachmann wählt die spezifische Ausgestaltung einer Dimethylether-Kohlendioxid-Destillationssäule (wie beispielsweise Art und Anzahl der Einbauten) und die Betriebsbedingungen (wie beispielsweise Betriebsdruck, Heiz- und Kühlfluide im Sumpfverdampfer und Kopfkondensator usw.) insbesondere auf Grundlage der Siedepunktsunterschiede von Dimethylether und Kohlendioxid aus.

Ein wesentlicher Aspekt der vorliegenden Erfindung ist die geeignete Einstellung der Betriebsbedingungen der Dimethylether-Kohlendioxid-Destillationssäule. Diese sind vorteilhafterweise derart gewählt, dass ein überwiegend Kohlendioxid enthaltendes Kopfgas, das vom Kopf der Dimethylether-Kohlendioxid-Destillationssäule abgezogen wird, oberhalb der Schmelztemperatur von Kohlendioxid verflüssigt werden kann. Ein entsprechend erhaltenes Kondensat sollte in ausreichender Menge zur Verfügung stehen, um einerseits als Rücklauf für die Dimethylether-Kohlendioxid-Destillationssäule verwendet zu werden und andererseits als Rücklauf für eine Absorptionskolonne zur Verfügung zu stehen, wie bereits teilweise erläutert.

Vorteilhafterweise wird die Dimethylether-Kohlendioxid-Destillationssäule derart betrieben, dass sich an ihrem Kopf ein überwiegend Kohlendioxid enthaltendes Kopfgas und in ihrem Sumpf eine an Dimethylether angereicherte Sumpfflüssigkeit bilden. Die Sumpfflüssigkeit enthält überwiegend Dimethylether, wenn die in die Dimethylether-Kohlendioxid-Destillationssäule eingespeisten Fluide (beispielsweise das oder die erläuterten Kondensate und das Sumpfprodukt aus der Absorptionssäule) überwiegend Kohlendioxid und Dimethylether enthalten. Enthalten letztere auch Wasser und Methanol, gehen diese ebenfalls in die Sumpfflüssigkeit der Dimethylether-Kohlendioxid-Destillationssäule über.

Die Erfindung ermöglicht damit durch die Verwendung des kohlendioxidreichen flüssigen Rücklaufs auf die Absorptionskolonne eine Verringerung der Verluste an Dimethylether. Als der überwiegend Kohlendioxid enthaltende Rücklauf wird dabei vorteilhafterweise ein Teil eines Kondensats verwendet, das aus zumindest einem Teil des Kopfgases der Dimethylether-Kohlendioxid-Destillationssäule gebildet wird. Sumpfseitig wird der Absorptionskolonne das bereits erwähnte flüssige Sumpfprodukt entnommen, das überwiegend aus Dimethylether und Kohlendioxid bestehen kann (und ggf. auch Wasser und Methanol aufweist). Vorzugsweise enthält dieses Sumpfprodukt den gesamten Dimethylether, der in die Absorptionskolonne eingespeist wurde oder dessen überwiegenden Anteil.

Die Abkühlung auf das zweite Temperaturniveau erfolgt vorteilhafterweise mittels vorhandener Kältemittel, beispielsweise C3- oder auch C2-Kältemittel (z.B. flüssigem Propan oder Ethan oder Äquivalenten zur Erreichung einer entsprechenden Temperatur). Eine vorige stufenweise Abkühlung auf Zwischentemperaturniveaus, falls durchgeführt, erfolgt beispielsweise mit Wasser und/oder einem C3-Kältemittel (z.B. flüssigem Propan oder einem Äquivalent). Dabei beträgt das erste Temperaturniveau vorteilhafterweise 20 bis 50 °C, insbesondere 30 bi s 40 °C. Das erste Temperaturniveau kann auch bei 50 bis 150 °C, insbe sondere bei 70 bis 120 °C, beispielsweise bei 80 bis 100 °C oder, auf den Taup unkt bezogen, beispielsweise mindestens 10 °C und maximal 30 bis 50 °C oberhalb des Taupunkts liegen. Das zweite Temperaturniveau liegt vorteilhafterweise zwischen der Schmelztemperatur von Kohlendioxid auf dem verwendeten Druckniveau und -15 °C, beispielsweise bei -40 bis -20 °C und insbesondere bei ca. -35 °C, der Tem peratur eines C3-Kältemittels. Das Temperaturniveau kann dabei auch knapp, d.h. mindestens 0,5 bis 10 °C, insbesondere 1 bis 5 °C, oberhalb der Schmelztemper atur von Kohlendioxid auf dem verwendeten Druckniveau liegen. Das verwendete Temperaturniveau richtet sich auch nach der Zusammensetzung des abgekühlten Gasgemischs und der gewünschten Zusammensetzung der abgeschiedenen Kondensate. Weil im Rahmen der vorliegenden Erfindung der kohlendioxidreiche Rücklauf aus entsprechenden Kondensaten gewonnen wird, wird das Temperaturniveau so gewählt, dass die Kondensate zumindest ausreichend Kohlendioxid enthalten. Der Kohlendioxidgehalt ist dann ausreichend, wenn bei der Auftrennung der Kondensate in der Dimethylether-Kohlendioxid-Destillationssäule an deren Kopf so viel Kohlendioxid erhalten wird, dass aus diesem ausreichend flüssiger Rücklauf auf die Dimethylether-Kohlendioxid-Destillationssäule und ausreichend flüssiger Rücklauf auf die Absorptionskolonne abgeschieden werden kann.

Das erfindungsgemäß vorgeschlagene Verfahren erweist sich als energetisch bedeutend günstiger als herkömmliche Verfahren, so dass durch die erfindungsgemäßen Maßnahmen eine vorteilhafte Trennung gegenüber Trennverfahren erzielt wird, wie sie aus dem Stand der Technik bekannt sind.

Die vorliegende Erfindung eignet sich insbesondere für Verfahren, bei denen der Produktstrom aus dem zur Synthese von Dimethylether aus Synthesegas verwendeten Reaktor auf einem Druckniveau von 20 bis 100 bar, insbesondere auf einem Druckniveau von 30 bis 80 bar (dem "ersten Druckniveau") bereitgestellt wird. Der Produktstrom kann, unter Verwendung einer weiteren Absorptionskolonne, auf diesem ersten Druckniveau weitgehend von Methanol und Wasser befreit werden. Die Abtrennung von Methanol und/oder Wasser kann also unter Druck erfolgen, eine vorherige Entspannung, die dann eine erneute energieaufwendige Druckbeaufschlagung erfordern würde, ist nicht notwendig. Das aus dem Produktstrom erhaltene Gasgemisch wird also nach dem Verlassen des Reaktors zur Synthese von Dimethylether und vor der erfindungsgemäßen trenntechnischen Bearbeitung nicht entspannt. Eine energieaufwendige erneute Verdichtung kann daher entfallen.

Die vorliegende Erfindung eignet sich für eine Trennung direkt im Anschluss an die Synthese und eine ggf. nachgeschaltete Abkühlung und/oder Entfernung von Wasser und/oder Methanol. Bei dieser liegt der Produktstrom bei dem erwähnten ersten Temperaturniveau vor.

Das erfindungsgemäße Verfahren kann mit Produktströmen unterschiedlichster Zusammensetzung zum Einsatz kommen. Entsprechende Produktströme enthalten beispielsweise 2 bis 50 Molprozent, insbesondere 5 bis 30 Molprozent Dimethylether, 0,1 bis 20 Molprozent, insbesondere 0,7 bis 10 Molprozent Methanol, 0,1 bis 20 Molprozent, insbesondere 0,8 bis 10 Molprozent Wasser, 1 bis 50 Molprozent, insbesondere 3 bis 30 Molprozent Kohlendioxid, 0,1 bis 25 Molprozent, insbesondere 1 bis 11 Molprozent Kohlenmonoxid und 5 bis 90 Molprozent, insbesondere 20 bis 80 Molprozent Wasserstoff. Wird eine Entfernung von Wasser und Methanol vorgenommen, ist das Gasgemisch vorzugsweise arm an Wasser und Methanol.

Entsprechende Produktströme können ferner geringere Anteile an weiteren Komponenten, beispielsweise Methan, Ethan, organische Säuren und höhere Alkohole enthalten, wie erwähnt. Entsprechende Gemische werden insbesondere in Verfahren erhalten, bei denen eine einstufige Synthese von Dimethylether vorgenommen wird.

Wie bereits erwähnt, wird die Dimethylether-Kohlendioxid-Destillationssäule im Rahmen der vorliegenden Erfindung vorteilhafterweise derart betrieben, dass ein überwiegend Kohlendioxid enthaltendes Kopfgas, das vom Kopf der Dimethylether-Kohlendioxid-Destillationssäule abgezogen wird, oberhalb der Schmelztemperatur von Kohlendioxid verflüssigt werden kann. Hierbei wird die Dimethylether-Kohlendioxid-Destillationssäule vorteilhafterweise auf einem zweiten Druckniveau betrieben, das unterhalb des ersten Druckniveaus liegt. Das zweite Druckniveau wird so gewählt, dass Kohlendioxid aus dem Kopfgas mit vorhandenen Kältemitteln möglichst weitgehend kondensiert werden kann. Die Temperatur solcher Kältemittel liegt typischerweise bei -52 bis -20 °C, beispielsweise b ei -35 °C (C3-Kältemittel). Die Sumpfflüssigkeit kann, wenn das eingesetzte Gasgemisch arm an Methanol und Wasser ist oder wenn Methanol und Wasser abgetrennt wurden, der Dimethylether-Kohlendioxid-Destillationssäule als dimethyletherreicher Strom entnommen werden, der einen Dimethylethergehalt von wenigstens 90 Molprozent, insbesondere wenigstens 95 Molprozent oder wenigstens 99 Molprozent aufweist. Die jeweiligen Gehalte richten sich nach den Betriebsbedingungen der Dimethylether-Kohlendioxid-Destillationssäule und deren Konfiguration. Sie können der jeweils geforderten Spezifikation (Reinheit) eines entsprechend erhaltenen Produktstroms angepasst werden. Enthält das Gasgemisch hingegen Methanol und Wasser in nennenswertem Umfang und/oder erfolgt keine Abtrennung von Methanol und Wasser, finden sich Methanol und Wasser in der Sumpfflüssigkeit wieder.

Wie erwähnt, besteht der flüssige Rücklauf, der auf die Absorptionskolonne aufgegeben wird, überwiegend aus Kohlendioxid. Vorteilhaft sind beispielsweise Kohlendioxidgehalte von wenigstens 90 Molprozent, insbesondere wenigstens 95 Molprozent oder wenigstens 99 Molprozent.

Das Verfahren ermöglicht eine Verringerung der Verluste an Dimethylether in der letzten Kondensationsstufe durch die Verwendung der Absorptionskolonne. Der aus dem Kopfgas der Dimethylether-Kohlendioxid-Destillationssäule gebildete, überwiegend Kohlendioxid enthaltende flüssige Rücklauf auf die Absorptionskolonne weist dabei vorteilhafterweise einen Dimethylethergehalt von höchstens 10 Molprozent, insbesondere höchstens 5 Molprozent, höchstens 1 Molprozent oder höchstens 0,5 Molprozent auf. Entsprechende Gehalte können auch im Kopfstrom der Absorptionskolonne erhalten werden.

Die Erfindung wird unter Bezugnahme auf die Zeichnungen näher erläutert, die eine Ausführungsform der Erfindung gegenüber dem Stand der Technik zeigen.

Kurze Beschreibung der Zeichnungen
Figur 1 zeigt eine Anlage zur Erzeugung von Dimethylether gemäß dem Stand der Technik in schematischer Darstellung
Figur 2 zeigt eine Anlage zur Erzeugung von Dimethylether gemäß einer Ausführungsform der Erfindung in schematischer Darstellung.

### Ausführliche Beschreibung der Zeichnungen

In den Figuren sind einander entsprechende Elemente mit identischen Bezugszeichen angegeben und werden der Übersichtlichkeit halber nicht wiederholt erläutert.

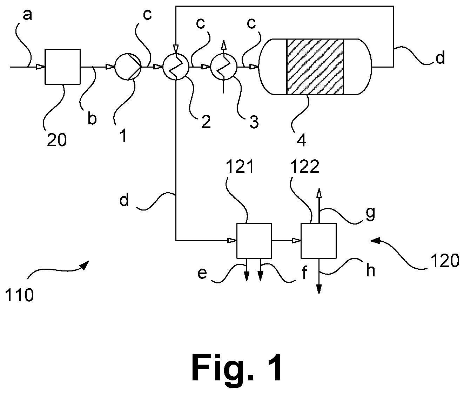

In Figur 1 ist eine Anlage, die ein Verfahren zur Erzeugung von Dimethylether gemäß dem Stand der Technik implementiert, schematisch dargestellt und insgesamt mit 110 bezeichnet.

Die Anlage 110 umfasst einen hier stark schematisiert dargestellten Synthesegasreaktor 20, der mit einem geeigneten Einsatz a, beispielsweise Erd- oder Biogas, beschickt werden kann. Dem Synthesegasreaktor 20 kann ein Synthesegasstrom b entnommen werden.

Der Synthesegasstrom b kann, gegebenenfalls nach einer Beimischung weiterer Ströme, mittels eines Verdichters 1 druckerhöht werden. Hierdurch kann ein für eine nachfolgende einschrittige Dimethylethersynthese erforderlicher Druck, beispielsweise ein Druck von 20 bis 100 bar, eingestellt werden.

Ein entsprechend verdichteter Strom, nun mit c bezeichnet, wird durch einen ersten Wärmetauscher 2 geführt, der mit einem Produktstrom d eines Reaktors 4 zur Synthese von Dimethylether (siehe unten) erwärmt werden kann. Der entsprechend erwärmte Strom, weiter mit c bezeichnet, weist stromab des ersten Wärmetauschers 2 beispielsweise eine Temperatur von 200 bis 300 °C a uf. Der Strom c wird gegebenenfalls durch einen zweiten Wärmetauscher 3 geführt, welcher auch als Spitzenerhitzer bezeichnet wird.

Der in dem zweiten Wärmetauscher 3 weiter erwärmte Strom, auch hier weiter mit c bezeichnet, wird in den Reaktor 4 eingespeist, der als Rohrreaktor ausgebildet ist und dessen Reaktionsrohre mit einem geeigneten Katalysator zur Einschrittsynthese von Dimethylether befüllt sind. Die Darstellung in der Figur 1 ist stark vereinfacht. Typischerweise sind Reaktoren 4 zur Synthese von Dimethylether stehend angeordnet, wobei ein Strom c bodenseitig in den Rohrreaktor 4 eingespeist wird. Kopfseitig wird dem Reaktor 4 ein Strom d entnommen.

Aufgrund der exothermen Reaktion in dem Rohrreaktor 4 liegt der Strom d bei nochmals höherer Temperatur vor. Der Strom d wird als Heizmedium durch den Wärmetauscher 2 geführt. Er kühlt sich hierdurch auf eine Temperatur ab, die beispielsweise ca. 30 °C oberhalb der Temperatur de s Stroms c stromab des Verdichters 1 liegt. Der entsprechend abgekühlte Strom, weiter mit d bezeichnet, wird einer herkömmlichen Trennanlage 120 zugeführt. In der Trennanlage 120 werden aus dem Strom d beispielsweise unter zwischenzeitlicher Entspannung, Abkühlung, Rückverdichtung usw. (nicht dargestellt) in einem Schritt 121 ein Methanolstrom e und ein Wasserstrom f abgetrennt. Aus dem verbleibenden Rest werden die Ströme g und h gebildet, bei denen es sich beispielsweise um einen an Kohlendioxid angereicherten Strom g und einen an Dimethylether angereicherten Strom h handeln kann.

Die Zusammensetzung der Ströme g und h richtet sich unter anderem nach der Zusammensetzung des Stroms d und der spezifischen Ausgestaltung und den Betriebsparametern der Trennanlage 120.

Figur 2 zeigt eine Anlage, die ein Verfahren zur Erzeugung von Dimethylether gemäß einer Ausführungsform der Erfindung implementiert. Diese ist insgesamt mit 100 bezeichnet.

Eine erste Absorptionskolonne, die zur Abtrennung von Methanol und/oder Wasser eingesetzt wird, ist in der Figur 2 mit 6 bezeichnet. Wie bereits erläutert unterscheidet sich eine Absorptionskolonne 6 von einer Destillationssäule wie der Destillationssäule 5 (siehe unten) unter anderem dadurch, dass sie keinen Sumpfverdampfer aufweist. In der Absorptionskolonne 6 aufsteigende Dämpfe werden durch einen am Kopf der Absorptionskolonne 6 aufgegebenen Rücklauf gewaschen, so dass sich am Kopf der Absorptionskolonne 6 die leichter flüchtigen und im Sumpf der Absorptionskolonne 6 die schwerer flüchtigen Komponenten anreichern.

In der Anlage 100, die in Figur 2 dargestellt ist, wird der Strom d in die Absorptionskolonne 6 eingeleitet. Vom Kopf der Absorptionskolonne 6 wird ein Kopfstrom k entnommen und in einem Wärmetauscher 7 gegen ein geeignetes Kältemittel, beispielsweise Kühlwasser, gekühlt. Der entsprechend abgekühlte Strom k wird in einen Abscheiderbehälter 8 überführt, aus dessen Sumpf ein flüssiger Strom I entnommen und mittels einer Pumpe (ohne Bezeichnung) zumindest zum Teil als Rücklauf auf die Absorptionskolonne 6 aufgegeben wird.

Enthält der Strom d im dargestellten Beispiel neben Dimethylether auch Methanol, Wasser, Kohlendioxid, Kohlenmonoxid und Wasserstoff (neben Spuren anderer Verbindungen wie oben erläutert), gehen hiervon aufgrund der erläuterten Rückwaschung Dimethylether, Kohlendioxid, Kohlenmonoxid und Wasserstoff in den Kopfstrom k über. Durch eine geeignete Abkühlung in dem Wärmetauscher 7 und entsprechende Abscheidebedingungen in dem Abscheiderbehälter 8 scheidet sich in dem Abscheiderbehälter 8 ein Sumpfprodukt ab, das im Wesentlichen aus Dimethylether und Kohlendioxid (gegebenenfalls mit Spuren von Methanol) besteht. Vom Kopf des Abscheiderbehälters 8 kann ein Strom m gasförmig abgezogen werden, der zusätzlich zu Kohlendioxid, Kohlenmonoxid und Wasserstoff ebenfalls noch Dimethylether enthält. Der Strom m wird anschließend einer sequenziellen Abkühlung und Kondensation unterworfen, wie unten erläutert. Der nicht als flüssiger Rücklauf auf die Absorptionskolonne 6 aufgegebene Anteil des Stroms I wird, wie die bei der sequenziellen Abkühlung und Kondensation des Strom m anfallenden Kondensate, in eine Destillationssäule 9, die hier als Dimethylether-Kohlendioxid-Destillationssäule 9 bezeichnet wird, eingespeist.

Es sei ausdrücklich betont, dass die spezifische Bereitstellung des Stroms k, der aus dem Produktstrom d gewonnen wird, nicht in der dargestellten Weise erfolgen muss. Auch andere Möglichkeiten zur Abtrennung von Wasser und/oder Methanol sind einsetzbar, sofern sie zur Erzeugung eines Gasgemischs auf dem zuvor erwähnten ersten Druckniveau und dem ersten Temperaturniveau und mit den angegebenen Gehalten an den einzelnen Komponenten führen.

Die Erfindung kann auch ohne eine Abtrennung von Wasser und Methanol realisiert werden. In diesem Fall ist sicherzustellen, dass aus den oben erläuterten Gründen sowohl Wasser als auch Methanol in dem abzukühlenden Gasgemisch enthalten sind, um die Frostschutzwirkung des Methanols auszunutzen und die korrosive Wirkung des trockenen Methanols zu vermeiden.

Aus dem Sumpf der Absorptionskolonne 6 wird ein flüssiger Strom n entnommen und in geeigneter Höhe in eine Destillationssäule 5 eingespeist, welche mit einem Sumpfverdampfer 51 und einem Kopfkondensator 52 betrieben wird. Der Strom n enthält im dargestellten Beispiel den überwiegenden Anteil des in dem Strom d enthaltenen Wassers und Methanols.

Der Sumpfverdampfer 51 und der Kopfkondensator 52 werden mit geeigneten, vorzugsweise in einer entsprechenden Anlage vorhandenen, Heiz- bzw. Kühlmedien betrieben. In dem Sumpfverdampfer 51 wird ein flüssiger, aus dem Sumpf der Destillationssäule 5 abgezogener Strom zum Teil verdampft und in einen unteren Bereich der Destillationssäule 5 eingespeist. Ein nicht verdampfter Anteil kann als Strom p abgezogen werden.

Vom Kopf der Destillationssäule 5 wird ein gasförmiger Strom abgezogen, zum Teil in dem Kopfkondensator 52 der Destillationssäule 5 verflüssigt und als flüssiger Rücklauf in einem oberen Bereich erneut in die Destillationssäule 5 eingespeist. Ein gasförmig verbleibender Anteil o wird abgezogen.

Aus dem Strom n, der im Wesentlichen noch Wasser, Methanol, Wasserstoff, Dimethylether und Kohlendioxid enthält, wird also in der Destillationssäule 5 ein im Wesentlichen Dimethylether und Kohlendioxid enthaltender Strom (Strom o) und ein im Wesentlichen Methanol und Wasser enthaltender Strom (Strom p) gebildet. Der Strom o kann an geeigneter Stelle in den Trennprozess zurückgeführt werden. Der Strom p kann anderweitig verwendet werden. Abgeschiedenes Wasser kann auch zu einer Abwasserbehandlung oder einer Entgasung geführt werden.

Rücklaufmenge und Bodenzahl der Absorptionskolonne 6 können so optimiert werden, dass ein entsprechendes Sumpfprodukt n in möglichst geringer Menge anfällt. Vorteilhafterweise wird der Rücklauf, der als Anteil des Stroms I auf die Absorptionskolonne 6 aufgebracht wird, derart eingestellt, dass der Methanol- und Wassergehalt in dem Strom k minimiert wird. Die sich auf diese Weise ergebende Zusammensetzung des Stroms m ist derart, dass sich in der Abkühlungs- und Kondensationssequenz, der der Strom m unterworfen wird, die zuvor erläuterten Nachteile nicht mehr einstellen können.

Die bereits mehrfach erwähnten Schritte zur Weiterbehandlung des Stroms m sind hier insgesamt mit 10 angegeben. Der Strom m wird dabei zunächst einem Wärmetauscher 11 zugeführt und anschließend in einen Abscheiderbehälter 12 eingespeist. Die Abkühlung in dem Wärmetauscher 11 wird dabei derart vorgenommen, dass sich in dem Abscheiderbehälter 12 ein erstes Kondensat q abscheidet. Ein in dem Abscheiderbehälter 12 gasförmig verbleibender Anteil wird einem Wärmetauscher 13 zugeführt und anschließend in einen weiteren Abscheiderbehälter 14 eingespeist. Auch dort wird ein Kondensat, hier mit r bezeichnet, erhalten.

Die Kondensate q und r werden, zusammen mit dem nicht auf die Absorptionskolonne 6 rückgeführten Anteil des Stroms I, in die bereits erwähnte Dimethylether-Kohlendioxid-Destillationssäule 9 eingespeist, die wie unten erläutert betrieben wird. Ein auch am Kopf des Abscheiderbehälters 14 gasförmig verbleibender Anteil wird in einem weiteren Wärmetauscher 15 abgekühlt. Er liegt stromab des Wärmetauschers 15 auf dem mehrfach erläuterten "zweiten" Temperaturniveau zwischen dem Schmelzpunkt von Kohlendioxid (bei dem vorherrschenden Druck) und -15 °C vor. Die Temperatur des Stroms m stromauf des Wärmetauschers 11 (also das "erste" Temperaturniveau) beträgt demgegenüber beispielsweise +35 °C. Der entsprechend abgekühlte Strom, hier mit s bezeichnet, wird in eine Absorptionskolonne 16 überführt, die erfindungsgemäß betrieben werden kann.

Die Erfindung kann auch in einer stark vereinfachten Anordnung zum Einsatz kommen, beispielsweise bei einer einstufigen Abkühlung und ohne eine Abtrennung von Methanol und Wasser. Immer wird jedoch ein auf dem zweiten Temperaturniveau gasförmig verbleibender Anteil des Gasgemischs in einer Absorptionskolonne 16 mit einem überwiegend Kohlendioxid enthaltenden Rücklauf gewaschen, wie nachfolgend erläutert. Der überwiegend Kohlendioxid enthaltende Rücklauf wird dabei aus einem bei der Abkühlung abgeschiedenen flüssigen Anteil des Gasgemischs gebildet.

Der Strom s enthält im dargestellten Beispiel noch Dimethylether, Kohlendioxid, Kohlenmonoxid und Wasserstoff, also neben Dimethylether und Kohlendioxid hier zwei leichter als Dimethylether siedende Komponenten. Unter Verwendung eines flüssigen Rücklaufs v, der reich an Kohlendioxid ist und aus einem Teil eines Kondensats u gebildet wird, das aus einem Kopfstrom t aus einem Kopfgas der Dimethylether-Kohlendioxid-Destillationssäule 9 erhalten wird, wird im Sumpf der Absorptionskolonne 16 ein Gemisch aus Dimethylether und Kohlendioxid abgeschieden und in Form des Sumpfprodukts w abgezogen. Das Sumpfprodukt w kann ebenfalls in die Dimethylether-Kohlendioxid-Destillationssäule 9 eingespeist werden. Am Kopf der Absorptionskolonne 16 wird hingegen ein Kopfprodukt x abgezogen, das im Wesentlichen aus Kohlendioxid, Kohlenmonoxid und Wasserstoff besteht und arm oder vorzugsweise frei an Dimethylether ist. Dieses kann, gegebenenfalls nach entsprechender Verdichtung in einem Verdichter 17, anderweitig verwendet werden.

In die Dimethylether-Kohlendioxid-Destillationssäule 9 werden, wie erwähnt, der nicht in die Absorptionskolonne 6 rückgeführte Anteil des Stroms I sowie die Ströme q und r und das Sumpfprodukt w eingespeist. Da diese unterschiedliche Gehalte an Dimethylether und Kohlendioxid aufweisen (Spuren von Kohlenmonoxid und Wasserstoff sind in gelöster Form ebenfalls enthalten), werden diese in unterschiedlicher Höhe in die Dimethylether-Kohlendioxid-Destillationssäule 9 eingespeist, wozu geeignete Ventile (ohne Bezeichnung) vorgesehen sind.

Auch die Dimethylether-Kohlendioxid-Destillationssäule 9 wird mit einem Sumpfverdampfer 91 und einem Kopfkondensator 92 betrieben. Ein aus einem Kopfgas der Dimethylether-Kohlendioxid-Destillationssäule 9 gebildeter Kopfstrom t wird in dem Kopfkondensator 92 unter Verwendung eines mit einem geeigneten Kältemittel betriebenen Wärmetauschers zumindest teilweise verflüssigt und als flüssiger Rücklauf auf die Dimethylether-Kohlendioxid-Destillationssäule 9 aufgegeben. Ein weiterer Anteil u wird zur Bildung des Rücklaufs v und eines weiteren Stroms y verwendet, der anderweitig verwendet werden kann.

Aus dem Sumpf der Dimethylether-Kohlendioxid-Destillationssäule 9 wird ein flüssiger Strom z entnommen, der hier im Wesentlichen aus Dimethylether besteht, jedoch insbesondere frei oder arm an Kohlendioxid ist.

## Patentansprüche

1. Verfahren zur trenntechnischen Bearbeitung eines Gasgemischs (k), das aus einem Produktstrom (d) eines Reaktors (4) zur Synthese von Dimethylether aus Synthesegas (b) gebildet wird, und das zumindest Dimethylether, Kohlendioxid und zumindest eine weitere, leichter als Kohlendioxid siedende Komponente enthält, wobei das Gasgemisch (k) auf einem ersten Druckniveau von einem ersten auf ein zweites Temperaturniveau abgekühlt wird und ein auf dem zweiten Temperaturniveau gasförmig bleibender Anteil des Gasgemischs (k) zur Entfernung von noch enthaltenem Dimethylether in einer Absorptionskolonne (16) mit einem Rücklauf (v) gewaschen wird, wobei der Rücklauf (v) zumindest teilweise aus einem bei der Abkühlung flüssig abgeschiedenen Anteil des Gasgemischs (k) gebildet wird, **dadurch gekennzeichnet, dass** der Rücklauf (v) ein überwiegend Kohlendioxid enthaltender Rücklauf ist.

2. Verfahren nach Anspruch 1, bei dem das Gasgemisch (k) über mehrere Zwischentemperaturniveaus auf das zweite Temperaturniveau abgekühlt wird, wobei mehrere Kondensate (l, q, r) abgeschieden werden.

3. Verfahren nach Anspruch 1 oder 2, bei dem der überwiegend Kohlendioxid enthaltende Rücklauf (v) teilweise aus einem Sumpfprodukt (w) der Absorptionskolonne (16) gebildet wird.

4. Verfahren nach einem der vorstehenden Ansprüche, bei dem der überwiegend Kohlendioxid enthaltende Rücklauf (v) unter Verwendung einer Dimethylether-Kohlendioxid-Destillationssäule (9) gebildet wird.

5. Verfahren nach Anspruch 4, bei dem die Dimethylether-Kohlendioxid-Destillationssäule (9) derart betrieben wird, dass sich an ihrem Kopf ein überwiegend Kohlendioxid enthaltendes Kopfgas (t) und in ihrem Sumpf eine an Dimethylether angereicherte Sumpfflüssigkeit bilden.

6. Verfahren nach Anspruch 5, bei dem als der überwiegend Kohlendioxid enthaltende Rücklauf (v) ein Teil eines Kondensats verwendet wird, das aus zumindest einem Teil des Kopfgases der Dimethylether-Kohlendioxid-Destillationssäule (9) gebildet wird.

7. Verfahren nach Anspruch 5 oder 6, bei dem zumindest ein Teil der Sumpfflüssigkeit der Dimethylether-Kohlendioxid-Destillationssäule (9) als Produktstrom (z) abgezogen wird, der einen Dimethylethergehalt von mehr als 90 Molprozent, insbesondere mehr als 95 Molprozent aufweist.

8. Verfahren nach einem der Ansprüche 4 bis 7, bei dem die Dimethylether-Kohlendioxid-Destillationssäule (9) auf einem zweiten Druckniveau betrieben wird, das unterhalb des ersten Druckniveaus liegt.

9. Verfahren nach einem der vorstehenden Ansprüche, bei dem der überwiegend Kohlendioxid enthaltende Rücklauf (v) einen Dimethylethergehalt von höchstens 10 Molprozent, insbesondere höchstens 5 Molprozent, höchstens 1 Molprozent oder höchstens 0,5 Molprozent aufweist.

10. Verfahren nach einem der vorstehenden Ansprüche, bei dem das Gasgemisch (k) durch zumindest teilweises Abtrennen von Methanol und/oder Wasser aus dem Produktstrom (d) gebildet wird.

11. Verfahren nach einem der vorstehenden Ansprüche, bei dem das erste Temperaturniveau 20 bis 50 °C, insbesondere 30 bis 40 °C beträgt und/oder bei dem das zweite Temperaturniveau zwischen der Schmelztemperatur von Kohlendioxid auf dem verwendeten Druckniveau und -15 °C liegt.

12. Verfahren nach einem der vorstehenden Ansprüche, bei dem das erste Druckniveau 20 bis 100 bar, insbesondere 30 bis 80 bar beträgt.

## Claims

1. Process for the separative treatment of a gas mixture (k) which is formed by a product stream (d) from a reactor (4) for the synthesis of dimethyl ether from synthesis gas (b) and contains at least dimethyl ether, carbon dioxide and at least one further component having a boiling point lower than that of carbon dioxide, where the gas mixture (k) is cooled at a first pressure level from a first temperature level to a second temperature level and a proportion of the gas mixture (k) remaining in gaseous form at the second temperature level is scrubbed with a runback (v) in an absorption column (16) to remove dimethyl ether still present, where the runback (v) is formed at least partly by a proportion of the gas mixture (k) separated off in liquid form on cooling, **characterized in that** the runback (v) is a runback containing predominantly carbon dioxide.

2. Process according to Claim 1, wherein the gas mixture (k) is cooled via a plurality of intermediate temperature levels to the second temperature level, with a plurality of condensates (l, q, r) separating out.

3. Process according to Claim 1 or 2, wherein the runback (v) containing predominantly carbon dioxide is formed partly by a bottom product (w) from the absorption column (16).

4. Process according to any of the preceding claims, wherein the runback (v) containing predominantly carbon dioxide is formed using a dimethyl ether-carbon dioxide distillation column (9).

5. Process according to Claim 4, wherein the dimethyl ether-carbon dioxide distillation column (9) is operated in such a way that an overhead gas (t) containing predominantly carbon dioxide is formed at the top thereof and a bottom liquid enriched in dimethyl ether is formed at the bottom thereof.

6. Process according to Claim 5, wherein part of a condensate formed from at least part of the overhead gas from the dimethyl ether-carbon dioxide distillation column (9) is used as the runback (v) containing predominantly carbon dioxide.

7. Process according to Claim 5 or 6, wherein at least part of the bottom liquid from the dimethyl ether-carbon dioxide distillation column (9) is taken off as product stream (z) which has a dimethyl ether content of more than 90 mol per cent, in particular more than 95 mol per cent.

8. Process according to any of Claims 4 to 7, wherein the dimethyl ether-carbon dioxide distillation column (9) is operated at a second pressure level which is below the first pressure level.

9. Process according to any of the preceding claims, wherein the runback (v) containing predominantly carbon dioxide has a dimethyl ether content of not more than 10 mol per cent, in particular not more than 5 mol per cent, not more than 1 mol per cent or not more than 0.5 mol per cent.

10. Process according to any of the preceding claims, wherein the gas mixture (k) is formed by at least partial separation of methanol and/or water from the product stream (d).

11. Process according to any of the preceding claims, wherein the first temperature level is from 20 to 50°C, in particular from 30 to 40°C, and/or the second temperature level is in the range from the melting point of carbon dioxide at the pressure level used to -15°C.

12. Process according to any of the preceding claims, wherein the first pressure level is from 20 to 100 bar, in particular from 30 to 80 bar.

## Revendications

1. Procédé de traitement de séparation d'un mélange gazeux (k), qui est formé par un courant de produits (d) d'un réacteur (4) pour la synthèse d'éther diméthylique à partir d'un gaz de synthèse (b), et qui contient au moins de l'éther diméthylique, du dioxyde de carbone et au moins un autre composant ayant un point d'ébullition plus bas que le dioxyde de carbone, le mélange gazeux (k) étant refroidi à un premier niveau de pression d'un premier à un deuxième niveau de température, et une fraction du mélange gazeux (k) qui reste sous forme gazeuse au deuxième niveau de température étant lavée pour l'élimination de l'éther diméthylique encore contenu dans une colonne d'absorption (16) avec un reflux (v), le reflux (v) étant formé au moins en partie par une fraction du mélange gazeux (k) séparée sous forme liquide lors du refroidissement, **caractérisé en ce que** le reflux (v) est un reflux contenant principalement du dioxyde de carbone.

2. Procédé selon la revendication 1, selon lequel le mélange gazeux (k) est refroidi par le biais de plusieurs niveaux de température intermédiaires au deuxième niveau de température, plusieurs condensats (l, q, r) étant séparés.

3. Procédé selon la revendication 1 ou 2, selon lequel le reflux (v) contenant principalement du dioxyde de carbone est formé en partie par un produit de fond (w) de la colonne d'absorption (16).

4. Procédé selon l'une quelconque des revendications précédentes, selon lequel le reflux (v) contenant principalement du dioxyde de carbone est formé en utilisant une colonne de distillation éther diméthylique-dioxyde de carbone (9).

5. Procédé selon la revendication 4, selon lequel la colonne de distillation éther diméthylique-dioxyde de carbone (9) est exploitée de sorte qu'un gaz de tête (t) contenant principalement du dioxyde de carbone se forme à sa tête et un liquide de fond enrichi en éther diméthylique dans son fond.

6. Procédé selon la revendication 5, selon lequel une partie d'un condensat qui est formé par au moins une partie du gaz de tête de la colonne de distillation éther diméthylique-dioxyde de carbone (9) est utilisée en tant que reflux (v) contenant principalement du dioxyde de carbone.

7. Procédé selon la revendication 5 ou 6, selon lequel au moins une partie du liquide de fond de la colonne de distillation éther diméthylique-dioxyde de carbone (9) est soutirée en tant que courant de produits (z), qui présente une teneur en éther diméthylique de plus de 90 pour cent en moles, notamment de plus de 95 pour cent en moles.

8. Procédé selon l'une quelconque des revendications 4 à 7, selon lequel la colonne de distillation éther diméthylique-dioxyde de carbone (9) est exploitée à un deuxième niveau de pression qui se situe en dessous du premier niveau de pression.

9. Procédé selon l'une quelconque des revendications précédentes, selon lequel le reflux (v) contenant principalement du dioxyde de carbone présente une teneur en éther diméthylique d'au plus 10 pour cent en moles, notamment d'au plus 5 pour cent en moles, d'au plus 1 pour cent en moles ou d'au plus 0,5 pour cent en moles.

10. Procédé selon l'une quelconque des revendications précédentes, selon lequel le mélange gazeux (k) est formé par séparation au moins partielle de méthanol et/ou d'eau du courant de produits (d).

11. Procédé selon l'une quelconque des revendications précédentes, selon lequel le premier niveau de température est de 20 à 50 °C, notamment de 30 à 40 °C et/ou selon lequel le deuxième niveau de température est compris entre la température de fusion du dioxyde de carbone au niveau de pression utilisé et -15 °C.

12. Procédé selon l'une quelconque des revendications précédentes, selon lequel le premier niveau de pression est de 20 à 100 bar, notamment de 30 à 80 bar.
